# EUROPEAN PATENT APPLICATION

(11) **EP 4 785 937 A1**
(43) Date of publication of application: **05.08.2026**
(21) Application number: 25155513.2
(22) Date of filing: 03.02.2025
(51) Int. Cl.: A61K 31/194, A61K 9/00, A61K 31/21, A61K 31/714, A61K 33/00, A61P 39/02

(54) **PHARMACEUTICAL COMPOSITION FOR CYANIDE POISONING**

(71) Applicant: Innotesto BV, 2460 Kasterlee (BE)
(72) Inventor: MERKUS, Fransiscus Wilhelmus, 2460 Kasterlee (BE)
(74) Representative: Arnold & Siedsma

(57) **Abstract**

A kit for the treatment of cyanide poisoning includes a pharmaceutical composition of hydroxocobalamin and/or pharmaceutically acceptable hydroxocobalamin salts. This composition is formulated for mucosal, nasal, or pulmonary administration. The kit may also contain additional therapeutic agents such as sodium thiosulfate, amyl nitrite, sodium nitrite, and 4-dimethylaminophenol. The pharmaceutical composition can be provided in single-dose or multi-dose containers, with instructions for use in a drug delivery device for effective treatment.

## Description

### TECHNICAL FIELD

The present disclosure relates to a pharmaceutical composition for the treatment of cyanide poisoning, and more particularly, to a kit comprising a pharmaceutical composition for mucosal, nasal, or pulmonary administration.

### BACKGROUND

Cyanide poisoning is a critical medical emergency that can result from exposure to cyanide compounds, which are highly toxic and can lead to rapid respiratory failure and death if not treated promptly. Cyanide can be encountered in various industrial settings, during fires involving certain materials, and even in some chemical warfare scenarios. The need for effective and rapid treatment options is paramount, as cyanide acts quickly to inhibit cellular respiration, leading to severe hypoxia and metabolic acidosis.

Traditional treatments for cyanide poisoning have included the use of intravenous antidotes, which, while effective, may not always be practical in emergency situations where immediate medical intervention is required. The administration of antidotes via intravenous routes can be challenging in pre-hospital settings or in situations where medical personnel are not immediately available. Therefore, there is a significant need for alternative administration routes that can provide rapid and effective treatment for cyanide poisoning, potentially improving outcomes by allowing for quicker intervention.

### SUMMARY

The present invention provides a kit for treating cyanide poisoning and includes a pharmaceutical composition containing hydroxocobalamin or its pharmaceutically acceptable salts. The composition being designed for administration through mucosal, nasal, or pulmonary routes.

Some examples of the kit may specifically focus on mucosal administration, allowing the composition to be applied directly to mucosal surfaces.

In some cases, the kit may be tailored for nasal administration, enabling the composition to be delivered through the nasal passages.

The kit can also be formulated for pulmonary administration, allowing the composition to be nebulized or inhaled into the lungs.

Hydroxocobalamin or its pharmacuetically acceptable salts, are chosen as vitamin B12, especially because hydroxocobalamin is a scavenger of cyanide. By using an administration of a hydroxocobalamin (and/or hydroxocobalamin as acetate, hydrochloride or sulfate) through mucosal, nasal or pulmonary routes, in particular through inhalation or nebulization, an immediate "rescue" treatment can be offered.

The pharmaceutical composition in the kit may be aqueous and can contain hydroxocobalamin or its salts in concentrations ranging from about 2% to about 10% by weight/volume.

Some examples of the kit may include pharmaceutically acceptable salts of hydroxocobalamin such as hydroxocobalamin acetate, hydroxocobalamin hydrochloride, or hydroxocobalamin sulfate.

The composition may also contain at least one pharmaceutically acceptable excipient, which can be selected from solvents, buffers, antioxidants, pH-adjusting agents, surfactants, complexing agents, stabilizers, and solubilizers.

In some instances, the composition may be in an isotonic aqueous buffer with a pH of 7.4, such as a phosphate buffered saline (PBS) and can be formulated as an aerosol for mucosal, nasal, or pulmonary administration.

The composition may further include mannitol in amounts ranging from about 3% to about 12% by weight/volume, with some examples specifying a range of about 7% to about 8%.

The kit may also contain one or more solvents, which can be selected from ethanol, glycerol, polyethylene glycol, alkoxypolyethylene glycol, and propylene glycol.

In some cases, the composition may include a solvent such as ethanol or propylene glycol in amounts ranging from about 3% to about 15% by volume, with specific examples focusing on ethanol in amounts from about 5% to about 15%.

The kit may further comprise at least one additional therapeutic agent to be used in combination with the pharmaceutical composition for treating cyanide poisoning.

Some examples of additional therapeutic agents may include sodium thiosulfate, amyl nitrite, sodium nitrite, alpha ketoglutarate and 4-dimethylaminophenol.

The pharmaceutical composition may be provided in either a single-dose or multi-dose container.

In some instances, the kit includes a single-dose container along with instructions for using the single-dose in a nebulizer for treating cyanide poisoning.

In an aspect of the invention it provides the pharmaceutical composition of hydroxocobalamin or its salts formulated for mucosal, nasal, or pulmonary administration for use in treating cyanide poisoning.

The composition for use in treating cyanide poisoning may be as defined in some examples of the claims, specifically those detailing the composition's formulation.

A method for treating cyanide poisoning may involve administering the pharmaceutical composition to a patient in need, with the composition being formulated for mucosal, nasal, or pulmonary administration.

The method of treatment may utilize the composition as defined in some examples of the claims, particularly those specifying the composition's formulation.

### DETAILED DESCRIPTION

This disclosure provides a kit designed for the treatment of cyanide poisoning, which includes a pharmaceutical composition formulated for mucosal, nasal, or pulmonary administration. The composition contains hydroxocobalamin and/or pharmaceutically acceptable hydroxocobalamin salts, which may serve as the active ingredient for treatment. The pharmaceutical composition can be delivered through various administration methods, such as mucosal, nasal, or pulmonary routes, to effectively treat cyanide poisoning. The kit may also include a method for administering the pharmaceutical composition to a patient in need, ensuring the treatment of cyanide poisoning. The pharmaceutical composition can be provided in a single-dose or multi-dose container, potentially with instructions for use. In an embodiment the pharmaceutical composition is used in a nebulizer, facilitating ease of administration. The kit may also comprise additional therapeutic agents to enhance the treatment efficacy.

According to an embodiment, the method of treating cyanide poisoning involves the administration of a pharmaceutical composition that contains hydroxocobalamin and/or pharmaceutically acceptable hydroxocobalamin salts. This composition being formulated for mucosal, nasal, or pulmonary administration. The administration to the patient may be facilitated by the kit, which may include the pharmaceutical composition. The formulation of the pharmaceutical composition may be designed to deliver the active ingredient effectively to treat cyanide poisoning. The administration method may be selected based on the specific needs of the patient and the desired route of administration.

Inhalation and nebulization allow deposition of the drug in the respiratory tract, e.g., mouth, throat, trachea, bronchi and alveoli. The smaller the particle size, the longer the particle will remain suspended in air and the farther down the respiratory tract the drug can be delivered. The difference between nebulizers and other methods of pulmonary installation are that patient cooperation is not required and the delivery of higher doses of medication is easier.

The pharmaceutical composition may be prepared to ensure that the active ingredient, hydroxocobalamin, is delivered efficiently to the above mentioned sites of action. The treatment method may be adaptable to various administration routes, allowing for flexibility in treating cyanide poisoning. The kit may provide the necessary components, such as a delivery device, to ensure that the pharmaceutical composition is administered correctly and effectively. The administration process may be optimized to enhance the therapeutic effect of the pharmaceutical composition in treating cyanide poisoning.

The delivery device may selected from an atomizer, nebulizer, inhaler, vaporizer, aerosolizer and the like. In some embodiments activating the delivery device it is possible that at least a portion of the hydroxocobalamin solution is sprayed into the user's mouth. The method may also include inhaling. The method may also include activating the delivery device while the user inhales. The method may also include loading the hydroxocobalamin product into the spray flacon (unit dose and multi dose), atomizer, nebulizer, inhaler, vaporizer, aerosolizer and other devices.

In a particular embodiment a nebulizer or an inhaler (commonly a metered-dose inhaler, or MDI, often with a spacer) can be used to deliver the pharmaceutical composition that contains hydroxocobalamin and/or pharmaceutically acceptable hydroxocobalamin salts in the treatment of the cyanide poisoning.

The pharmaceutical composition comprises hydroxocobalamin and/or pharmaceutically acceptable hydroxocobalamin salts, which may be selected from hydroxocobalamin acetate, hydroxocobalamin hydrochloride, and hydroxocobalamin sulfate. This selection provides the active ingredient necessary for the treatment of cyanide poisoning. The composition may be formulated as an aqueous pharmaceutical composition, which may facilitate the delivery of the active ingredient. The formulation may be designed to ensure that the hydroxocobalamin and its salts are present in a concentration that is effective for treatment. The composition is prepared in such a way that it can be administered through mucosal, nasal, or pulmonary routes, which enhances the flexibility of administration based on patient needs. The inclusion of pharmaceutically acceptable excipients, solvents, and mannitol may further optimize the composition for stability, solubility, and patient comfort. The composition may be provided in a single-dose or multi-dose container, which may allow for ease of use and accurate dosing. The kit may also include instructions for use, which may guide the administration process to ensure effective treatment.

The pharmaceutical composition may further comprise at least one pharmaceutically acceptable excipient, which can be selected to enhance the stability or solubility of the active ingredient. The inclusion of one or more solvents may be considered to facilitate the dissolution or dispersion of the pharmaceutical composition. Mannitol may be included as an additional component, potentially serving as a stabilizer or bulking agent and facilitating the mucosal absorption of hydroxocobalamin and its salts. Mannitol (in a hypertonic concentration) is not only providing the consistent mucosal absorption of hydroxocobalamin, it also increases the fluidity of the mucus supporting and also restoring the normal mucociliary clearance, making mannitol the preferred excipient to facilitate an effective nasal/pulmonary absorption of hydroxocobalamin and its salts. The pharmaceutical composition may be provided in a single-dose or multi-dose container, which can be designed to ensure accurate dosing and ease of administration. The packaging of the pharmaceutical composition may be configured to maintain the integrity and efficacy of the formulation. The selection of excipients and solvents may be tailored to the specific requirements of the pharmaceutical composition, ensuring compatibility and effectiveness. The potential inclusion of mannitol may contribute to the overall formulation by providing additional benefits such as improved texture or stability. The container design may be optimized to facilitate the administration process, potentially enhancing patient compliance and treatment outcomes.

According to an embodiment, the kit may include at least one additional therapeutic agent, which can be used in conjunction with the pharmaceutical composition for the treatment of cyanide poisoning. This additional therapeutic agent may be selected from a group that includes sodium thiosulfate, alpha ketoglutarate, amyl nitrite, sodium nitrite, and 4-dimethylaminophenol. The kit may also comprise a single-dose container of the pharmaceutical composition, along with instructions detailing how the single-dose should be utilized for effective treatment. The inclusion of these additional therapeutic agents may enhance the treatment efficacy by providing a multi-faceted approach to counteracting cyanide poisoning. The instructions provided may ensure that the administration of the pharmaceutical composition is conducted accurately, potentially optimizing the therapeutic outcome. The single-dose container may facilitate ease of use and ensure the correct dosage is administered, which can be critical in emergency situations. The potential for the kit to include these components and instructions may offer a comprehensive solution for the treatment of cyanide poisoning, addressing both the immediate need for treatment and the practical aspects of administration.

In an embodiment of the invention the pharmaceutical compositions of the present invention are designed for administration by a nebulizer. A nebulized solution is one dispersed in air to form an aerosol, and a nebulizer generates very fine liquid droplets suitable for inhalation into the lung. Nebulizers typically use compressed air, ultrasonic waves, or a vibrating mesh to create a mist of the droplets and may also have a baffle to remove larger droplets from the mist by impaction. A variety of nebulizers are available for this purpose, such as ultrasonic nebulizers, jet nebulizers and breath-actuated nebulizers. In use, mouthpieces or masks are typically attached to a patient to aid delivery of the nebulized solution.

In a particular embodiment of the invention, formulations are for delivery with and patients are treated using a high efficiency nebulizer, in particular one that can deliver at least 15 %, preferably at least 25 %, more preferably at least 35% of the drug substance to the patient's lungs.

In specific embodiments of the invention, formulations are delivered using a high efficiency jet nebulizer, a high efficiency ultrasonic nebulizer or a high efficiency vibrating mesh nebulizer, use of these devices enabling and / or enhancing the use of the reduced volume formulations of the invention. Jet nebulizers are particularly preferred, and one example is the PARI LC Plus (registered trade mark, Pari GmbH, Germany) nebulizer.

### EXAMPLES

It is an object of the invention to provide methods of administering, and a formulation for administering hydroxocobalamin and/or hydroxocobalamin acetate, hydroxocobalamin hydrochloride and hydroxocobalamin sulfate, by inhalation or nebulization. The invention also relates to methods of treating a patient in need of hydroxocobalamin treatment, especially after a CN (cyanide) intoxication.

Hydroxocobalamin is soluble in water (about 1:50) and hydroxocobalamin as sulfate, as acetate and as hydrochloride is soluble in water 1:10 to 1:30 w/w (source: Martindale: The Complete Drug Reference 35thEdition, S. Sweetman, Pharmaceutical Press, 2007, page 1817 Vitamin B12 substances). Possible formulations of aqueous pharmaceutical compositions for administering hydroxocobalamin and/or hydroxocobalamin acetate, hydroxocobalamin hydrochloride and hydroxocobalamin sulfate, by inhalation or nebulization are provided in the examples hereinafter.

For each of the below examples the dose and volume of the solution used for nebulization were chosen on the basis of earlier studies and previous clinical works reported for other nebulized compounds. A fill volume of 4 ml was chosen with a nebulization time of 10min.

**Example 1:** nebulization and/or inhalation of liquid below
Liquid formulation of Hydroxocobalamin compound 2-5%
0.9% saline 70-90% % v/v
ethanol 10-30% v/v
The formulation is filtered through a 0.22-micron filter to make it sterile. The pH is set at 7.4.

**Example 2:** nebulization and/or inhalation of liquid below
Liquid formulation of Hydroxocobalamin compound 2-5%,
Mannitol 7%-8% w/v
Ethanol 5-15% v/v
0.9% Saline 85-95% v/v
The formulation is filtered through a 0.22-micron filter to make it sterile. The pH is set at 7.4.

**Example 3:** nebulization and/or inhalation of liquid below
Liquid formulation of Hydroxocobalamin compound 2-10 %,
Mannitol 7%-8%
Cyclodextrin derivative qs .
0.9% Saline qs
Aqua ad 100%
The formulation is filtered through a 0.22-micron filter to make it sterile. The pH is set at 7.4.

Note: In examples 2 and 3 mannitol is added to the formulation. The presence of mannitol increases the osmolarity of the formulation and causes the composition to be hyperosmotic to the epithelial cells. To create an osmotic equilibrium, water leaves the epithelial cells, improves the fluidity of the mucus layer, and causes the epithelial cells to shrink, at the same time stretching the tight junctions between the cells. Stretching means that newly formed gaps stay open for a short period of time enabling diffusion of hydroxocobalamin through the gaps between epithelial cells into the systemic blood circulation.

## Claims

1. A kit for the treatment of cyanide poisoning, comprising a pharmaceutical composition of hydroxocobalamin and/or pharmaceutically acceptable hydroxocobalamin salts, wherein the pharmaceutical composition is formulated for mucosal, nasal or pulmonary administration.

2. The kit according to claim 1, wherein the pharmaceutical composition is formulated for mucosal administration.

3. The kit according to claim 1, wherein the pharmaceutical composition is formulated for nasal administration.

4. The kit according to claim 1, wherein the pharmaceutical composition is formulated for pulmonary administration.

5. The kit according to any one of claims 1-4, wherein the pharmaceutical composition is an aqueous pharmaceutical composition comprises hydroxocobalamin and/or pharmaceutically acceptable hydroxocobalamin salts in an amount ranging from about 2% (w/v) to about 10% (w/v).

6. The kit according to any one of claims 1-5, wherein the pharmaceutically acceptable hydroxocobalamin salts are selected from hydroxocobalamin acetate, hydroxocobalamin hydrochloride and hydroxocobalamin sulfate.

7. The kit according to any one of claims 1-6, wherein the pharmaceutical composition further comprises at least one pharmaceutically acceptable excipient, selected from solvents, buffers, antioxidants, agents to adjust the pH, surfactants, complexing agents, stabilizers and solubilizers.

8. The kit according to any one of claims 1-7, wherein the pharmaceutical composition of hydroxocobalamin and/or pharmaceutically acceptable hydroxocobalamin salts is in an aqueous buffer at a pH of 7.4 in an aerosol formulation for mucosal, nasal or pulmonary administration .

9. The kit according to any one of claims 1-8, wherein the pharmaceutical composition of hydroxocobalamin and/or pharmaceutically acceptable hydroxocobalamin salts further comprises mannitol in an amount ranging from about 3% (w/v) to about 12% (w/v); in particular in a range from about 7% (w/v) to about 8% (w/v).

10. The kit according to any one of claims 1-9, wherein the pharmaceutical composition of hydroxocobalamin and/or pharmaceutically acceptable hydroxocobalamin salts further comprises one or more solvents selected from ethanol, glycerol, polyethylene glycol, alkoxypolyethylene glycol, and propylene glycol.

11. The kit according to claim 10, wherein the pharmaceutical composition of hydroxocobalamin and/or pharmaceutically acceptable hydroxocobalamin salts further comprises a solvent selected from ethanol and propylene glycol in an amount ranging from about 3% (v/v) to about 15% (v/v); in particular ethanol in an amount ranging from about 5% (v/v) to about 15% (v/v).

12. The kit according to any one of claims 1-11, further comprising at least one additional therapeutic agent for use in combination with the pharmaceutical composition for treating cyanide poisoning.

13. The kit according to claim 12, wherein the at least one additional therapeutic agent is selected from the group consisting of sodium thiosulfate, alpha ketoglutarate, amyl nitrite, sodium nitrite, and 4-dimethylaminophenol.

14. The kit according to any one of claims 1-13, wherein the pharmaceutical composition is provided in a single-dose or multi-dose container.

15. The kit according to claim 14, wherein the kit comprises the pharmaceutical composition in a single-dose container and - instructions on how the single-dose is to be used in a nebulizer for treatment of cyanide poisoning.

16. A pharmaceutical composition of hydroxocobalamin and/or pharmaceutically acceptable hydroxocobalamin salts, wherein the pharmaceutical composition is formulated for mucosal, nasal or pulmonary administration; for use in the treatment of cyanide poisoning.

17. The pharmaceutical composition of hydroxocobalamin and/or pharmaceutically acceptable hydroxocobalamin salts for use according to claim 16, wherein said pharmaceutical composition is as defined in any one of claims 5 to 11.

18. A method of treating cyanide poisoning in a patient in need thereof, comprising administering to said patient a pharmaceutical composition of hydroxocobalamin and/or pharmaceutically acceptable hydroxocobalamin salts, wherein the pharmaceutical composition is formulated for mucosal, nasal or pulmonary administration.

19. The method according to claim 18, wherein said pharmaceutical composition is as defined in any one of claims 5 to 11.
